# EUROPEAN PATENT SPECIFICATION

(11) **EP 2 590 943 B1**
(45) Date of publication and mention of the grant of the patent: **01.10.2014**
(21) Application number: 11735614.7
(22) Date of filing: 04.07.2011
(51) Int. Cl.: C07D 207/16, C07C 35/37

(54) **PROCESS AND INTERMEDIATES FOR PREPARATION OF AN ACTIVE INGREDIENT**
VERFAHREN SOWIE ZWISCHENPRODUKTE ZUR HERSTELLUNG AKTIVER BESTANDTEILE
PROCÉDÉ ET INTERMÉDIAIRES POUR LA PRÉPARATION D'UN PRINCIPE ACTIF

(30) Priority: 06.07.2010 IT MI20101239
(43) Date of publication of application: 15.05.2013
(73) Proprietor: Chemelectiva Srl., 28100 Novara (IT)
(72) Inventor: BARATELLA, Marco, I-28100 Novara (IT)
(74) Representative: Longoni, Alessandra
(86) International application number: PCT/EP2011/061178
(87) International publication number: WO 2012/004210

(56) References cited:
- WO-A2-2008/084383
- VILLHAUER E B ET AL: "1-ÄÄ(3-HYDROXY-1-ADAMANTYL)AMINOÜACETYLÜ- 2-CYANO-(S)-PYRROLIDINE: A POTENT, SELECTIVE, AND ORALLY BIOAVAILABLE DIPEPTIDYL PEPTIDASE IV INHIBITOR WITH ANTIHYPERGLYCEMIC PROPERTIES", JOURNAL OF MEDICINAL CHEMISTRY, AMERICAN CHEMICAL SOCIETY, WASHINGTON, US, vol. 46, no. 13, 1 January 2003 (2003-01-01), pages 2774-2789, XP001165747, ISSN: 0022-2623, DOI: DOI:10.1021/JM030091L cited in the application
- SINGH SANTOSH KUMAR ET AL: "Synthesis of (S)-1-(2-chloroacetyl)pyrrolidine-2-carbon itrile: A key intermediate for dipeptidyl peptidase IV inhibitors", BEILSTEIN JOURNAL OF ORGANIC CHEMISTRY, BIOMED CENTRAL, LONDON, GB, vol. 4, no. 20, 12 June 2008 (2008-06-12), XP009146853, ISSN: 1860-5397, DOI: DOI:10.3762/BJOC.4.20 [retrieved on 2008-06-12]

## Description

The present invention relates to a process for the preparation of a pharmaceutical active ingredient and particularly relates to a process for the preparation of vildagliptin and useful intermediates of said process.

Vildagliptin, chemically known as (2S)-1-[2-[(3-hydroxy-1-adamantyl)amino]-acetyl]pyrrolidine-2-carbonitrile, is a compound of formula 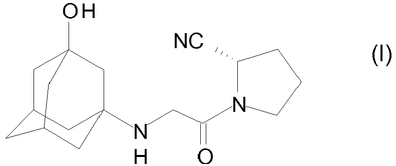 and is used in the therapeutic treatment of type 2 diabetes.

Vildagliptin is an orally active dipeptidylpeptidase IV (DPP IV) inhibitor and is described for the first time in WO 00/034241.

Various vildagliptin syntheses are known in the literature.

WO 00/034241 (Novartis AG) discloses a process comprising the reaction of a 2-cianopyrrolidine derivative of formula 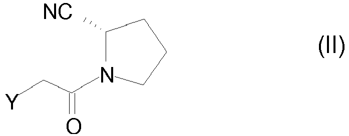 wherein Y represents a reactive group such as chloride, bromide or iodide; with 1-amino-3-adamantanol 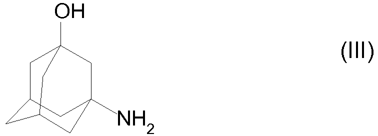 in the presence of a base and an inert solvent.

A similar synthesis is described in J. Med. Chem. 2003, 46, 2774-2789.

WO 2004/092127 (Novartis AG) discloses a process which does not includes the isolation of 2-cianopyrrolidine derivative (II), a particularly irritating intermediate, which reacts directly with 1-amino-3-adamantanol (III) to provide vildagliptin.

WO 2008/084383 (Medichem SA) discloses a process comprising the reaction of a 2-cianopyrrolidine derivative of formula (II) with a 1-amino-3-adamantanol wherein the OH group is protected..

WO 2010/022690 (Zentiva KS) discloses a process for the preparation of vildagliptin comprising the isolation of a 1-haloacetyl-2(S)-pyrrolidin-carboxyamide of formula 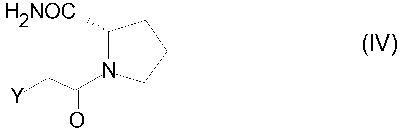 with a trialkylamine salt and its subsequent conversion into its corresponding ciano derivative of formula (II) which reacts with 1-amino-3-adamantanol following the conventional process of synthesis.

All known processes for the preparation of vildagliptin are characterized by the reaction of a derivative of formula (II) with an optionally protected 3-amino-1-adamantanol (III).

Furthermore the processes disclosed in WO 2008/084383 and WO 00/034241 lead to the (N, N)-alkylation compound of formula 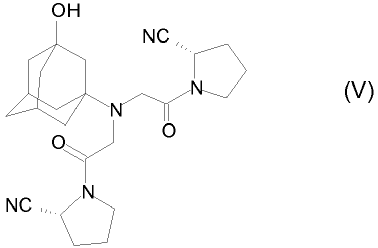 which causes difficulties in the purification step and reduction in product yield and purity.

Therefore there is a need for an alternative process for the synthesis of vildagliptin to provide said compound in good yield and high optical purity without involving the irritating intermediates of the known processes.

We have now found a process, which is an object of the present invention, for the synthesis of vildagliptin comprising the following steps:
a) the reaction of 1-amino-3-adamantanol (III) with glyoxylic acid to provide the intermediate of formula 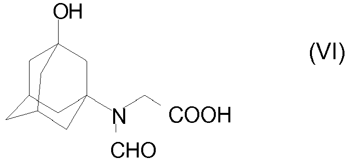
b) the reaction of intermediate (VI) with L-prolinamide to give the intermediate of formula 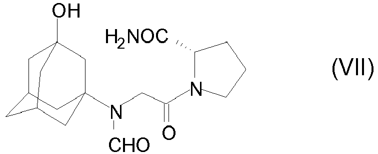
c) the dehydration of intermediate (VII) to give the corresponding nitrile derivative of formula 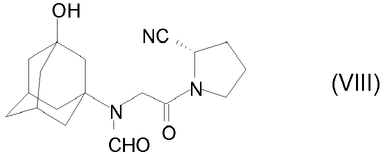
d) the deformylation of intermediate (VIII) to obtain vildagliptin (I).

The process object of the present invention is characterized by a synthesis pathway founded on new intermediates, completely different from the synthesis pathways of the known processes, which allows to obtain vildagliptin in good yield and high optical purity, avoiding the drawbacks due to the involvement of 2-cianopyrrolidin derivatives of formula (II) according to known state of the art.

The step a) of the process object of the present invention provides for the reaction of 1-amino-3-adamantanol (III) with glyoxylic acid in a suitable solvent at a temperature of from 0°C to 100°C; preferably from 40°C to 80°C.

Examples of suitable solvents are water, acetone, and methanol.

Water is preferably used.

Glyoxylic acid is generally used in excess, preferably in a double molar amount with respect to compound (III).

The reaction comprises the formation of the non-isolated intermediate of formula

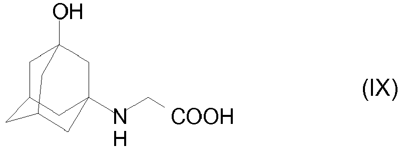

The reaction temperature is preferably from 20°C to 30°C.

At the end of the reaction the compound of formula 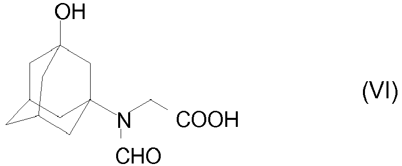 is obtained and the intermediate (VI) is new and is a further object of the present invention.

The intermediate (VI) is obtained as a crude through conventional isolation treatments of the reaction mixture, preferably through treatment with acetone. The crude (VI) can be used as such in the subsequent reaction b) or can be purified before the subsequent step through crystallization in a suitable solvent, preferably in water.

The step b) of the process object of the present invention provides for a coupling reaction between intermediate (VI) and L-prolinamide in the presence of a condensation agent and an aprotic solvent.

Suitable condensation agents are selected from carbonyldiimidazole, dicyclohexylcarbodiimide, 1,4,6-tri-n-propyl-2,4,6-trioxo-1,3,5,2,4,6-trioxa-triphosphorinane. Preferably carbonyldiimidazole is used.

Suitable aprotic solvents are acetonitrile and ethers.

Acetonitrile is preferably used.

The reaction is generally carried out at a temperature of from 0°C to 40°C, preferably from 20°C to 30°C.

At the end of the step b) the compound of formula 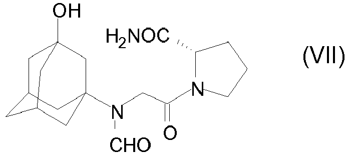 is obtained and this new intermediate is a further object of the present invention. The step c) of the process object of the present invention provides for the dehydration of intermediate (VII) to give the corresponding ciano derivative of formula

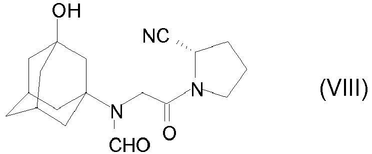

The reaction is carried out in the presence of a suitable dehydrating agent, such as an anhydride, in the presence of a base in an inert organic solvent.

Trifluoroacetic anhydride is preferably used as dehydrating agent.

Example of suitable bases are triethylamine, diethylisopropylamine, N,N-dimethylaminopyridine and ethylnicotinate; triethylamine is preferably used. Suitable solvents are dichloromethane, methyl-tert-butyl ether, tetrahydrofurane, and 2-methyl-tetrahydrofurane.

The reaction is preferably carried out in trifluoroacetic anhydride and triethylamine in dichloromethane at a temperature of from 0°C to 25°C, preferably of from 5°C to 10°C.

The intermediate of formula (VIII) is new and is a further object of the present invention.

The step d) of the process object of the present invention provides for the deformylation reaction of intermediate (VIII) to give vildagliptin (I).

Preferably the deformylation reaction is carried out by treatment with an acid and at warm, followed by the optional subsequent treatment with a base to obtain vildagliptin free base.

The acid used can be different depending on the vildagliptin salt to obtain. Generally, due to economic and practical reasons, the deformylation of the intermediate is preferably carried out in a solution of chloridric acid in water, followed by a treatment with soda.

The deformylation reaction is carried out by heating, generally at temperature of from 50°C to 80°C, preferably at about 70°C.

The resultant product can be optionally purified according to conventional techniques, preferably through crystallization.

At the end of the process object of the present invention vildagliptin is obtained in good yield and with optical purity and specification requirements suitable for its formulation in a pharmaceutical composition.

A preferred practical embodiment of the process object of the present invention is the following.

1-amino-3-adamantol (II) and an excess of glyoxylic acid are reacted in water at a temperature of from 20°C to 30°C.

At the end of the reaction the compound of formula 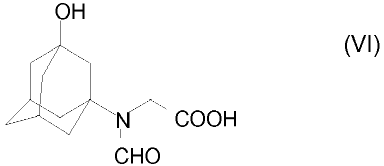 is obtained and isolated from the reaction mixture by treatment with acetone and purified through crystallization from water.

The intermediate (VI) is reacted with L-prolinamide in the presence of carbonyldiimidazole in acetonitrile medium at a temperature of from 20°C to 30°C. At the end of the reaction the compound of formula 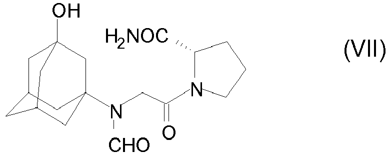 is obtained and then dehydrated to give the corresponding ciano derivative of formula 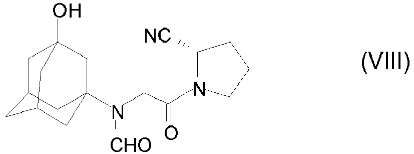 by treatment with trifluoroacetic anhydride and triethylamine in dichloromethane at a temperature of from 5°C to 10°C.

In said reaction the compound of formula 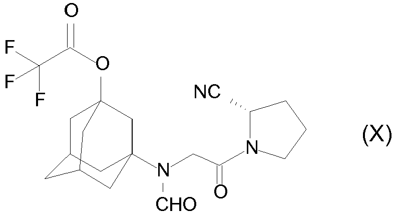 which is new and is a further object of the present invention, can be obtained. Particularly the intermediate (X) can be prepared in high yield by treating the compound of formula (VII) with trifluoroacetic anhydride in the absence of triethylamine.

Then the intermediate (VIII) is deformylated by treatment with hydrochloric acid in water at about 70°C followed by treatment with soda to obtain vildagliptin (I).

In order to better illustrate the present invention without limiting it, the following examples are now given.

### Example 1

500 ml of water and 221,3 g of an aqueous solution of glyoxylic acid 50 % (1,49 mol) were charged in a reaction flask. To the resultant solution, 100 g of 1-amino-3-adamantanol (0.60 mol) were gradually added, keeping the temperature from 20 °C to 30 °C. The reaction mixture was kept in such conditions for 48 hours. After completion of the reaction, water was distilled under vacuum to obtain a solid mass, which was loaded in a reaction flask containing 600 ml of acetone. The mixture was kept under stirring for 3 hours at a temperature of 30°C - 40°C, obtaining a white crystalline solid, which was crystallized from 700 ml of water, filtered and dried in oven to give 95 g of N-formyl-N-(3-hydroxy-1-adamantyl)aminoacetic acid.
¹H-NMR (D₂O, 300 MHz): δ 8.32 (s, 1 H), 4.09 (s, 2H), 2.28 (bs, 2H), 1.80 (m, 6H), 1.63 (m, 4H), 1.49 (bs, 2H).
¹³C-NMR (D₂O, 300 MHz): δ 173.1(C), 164.1 (CH), 69.6 (C), 59.7 (C), 47.2 (CH₂), 42.2 (CH₂), 41.9 (CH₂), 39.1 (CH₂), 33.6 (CH₂), 30.1 (CH)
ESI/MS (MH⁺): 254

### Example 2

207 g of 1-amino-3-adamantanol (1,23 mol) and 558 ml of water were charged in a reaction flask; the resultant suspension was heated to 70°C till complete solubilization. 552 g of glyoxylic acid 50 % in water (3,72 mol) were added dropwise in 2-hour time; at the end of the addition, the mixture was kept under stirring at 70°C for 4 hours. At the end of the reaction, the suspension was cooled at 20°C - 25°C and filtered washing with 2 x 100 ml of water, to give a solid mass which was crystallized from an acetone-water solution (1:2), dried under vacuum to give 159 g of N-formyl-N-(3-hydroxy-1-adamantyl)aminoacetic acid.
¹H-NMR (D₂O, 300 MHz): δ 8.32 (s, 1 H), 4.09 (s, 2H), 2.28 (bs, 2H), 1.80 (m, 6H), 1.63 (m, 4H), 1.49 (bs, 2H).
¹³C-NMR (D₂O, 300 MHz): δ 173.1(C), 164.1 (CH), 69.6 (C), 59.7 (C), 47.2 (CH₂), 42.2 (CH₂), 41.9 (CH₂), 39.1 (CH₂), 33.6 (CH₂), 30.1 (CH)
ESI/MS (MH⁺): 254

### Example 3

100 g of N-formyl-N-(3-hydroxy-1-adamanthyl)aminoacetic acid (0,39 mol), prepared as described in examples 1 or 2, were charged in a reaction flask under nitrogen flow with 750 ml of acetonitrile and 48 g of triethylamine (0.47 mol); the reaction mixture was kept under stirring at room temperature till complete dissolution, then 45.5 g of L-prolinamide (0,40 mol) were added. 478 g of 1,4,6-tri-n-propyl-2,4,6-trioxo-1,3,5,2,4,6-trioxa-triphosporinane 50 % in acetonitrile (0,76 mol) were added to the obtained suspension, keeping the temperature from 20°C to 30°C. At the end of the addition the mixture was kept in such conditions for 3 hours. 160 g of potassium carbonate 50 % in water were added and the resultant solid was filtered. The mother liquors of filtration were concentrated under vacuum to remove the organic solvent and the resultant aqueous solution was diluted with 200 ml of water and the product was recovered by extraction with 3 x 200 ml of 1-butanol.

The combined organic layers were concentrated under vacuum to afford 130 g of N-formyl-N-(3-hydroxy-1-adamantyl)acetyl-pyrrolidine-2-amide as oily residue.
¹H-NMR (D₂O, 300 MHz): δ 8.25 (s, 1 H), 4.24 (t, 1 H), 4.09 (s, 2H), 3.53 (t, 2H), 2.19 (s, 2H), 2.13 (s, 2H), 1.88 (m, 2H), 1.80 (s, 6H), 1.55 (s, 4H), 1.41 (s, 2H)
¹³C-NMR (D₂O, 300 MHz): δ 169.5 (CH), 164.6 (C), 71.6 (C), 62.4 (CH), 61.5 (C), 49.4 (CH₂), 48.9 (CH₂), 44.4 (CH₂), 44.2 (CH₂), 41.2 (CH₂), 35.6 (CH₂), 32.1 (CH), 31.4 (CH₂), 26.3 (CH₂)
ESI/MS (MH⁺): 350

### Example 4

In a reaction flask under nitrogen flow, 100 g of N-formyl-N-(3-hydroxy-1-adamantyl)aminoacetic acid (0.39 mol), prepared as described in example 1 or 2, were suspended in 750 ml of acetonitrile. To the suspension, 68.2 g of N-N'-carbonyldiimidazole (0.43 mol) were added at 25°C and stirred to give a clear solution. To the solution, 44.9 g of L-prolinamide (0.39 mol) were added and solubilized during the reaction proceeding. The reaction mixture was kept at 25°C for 5 hours, then concentrated in vacuum up to obtain a solid residue which was recovered in acetone, filtered and dried in oven under vacuum, obtaining 103 g of N-formyl-N-(3-hydroxy-1-adamantyl)acetyl-pyrrolidine-2-amide as a solid.
¹H-NMR (D₂O, 300 MHz): δ 8.25 (s, 1 H), 4.24 (t, 1 H), 4.09 (s, 2H), 3.53 (t, 2H), 2.19 (s, 2H), 2.13 (s, 2H), 1.88 (m, 2H), 1.80 (s, 6H), 1.55 (s, 4H), 1.41 (s, 2H)
¹³C-NMR (D₂O, 300 MHz): δ 169.5 (CH), 164.6 (C), 71.6 (C), 62.4 (CH), 61.5 (C), 49.4 (CH₂), 48.9 (CH₂), 44.4 (CH₂), 44.2 (CH₂), 41.2 (CH₂), 35.6 (CH₂), 32.1 (CH), 31.4 (CH₂), 26.3 (CH₂)
ESI/MS (MH⁺): 350

### Example 5

In a reaction flask under nitrogen flow, 100 g of N-formyl-N-(3-hydroxy-1-adamantyl)acetic-pyrrolidine-2-amide (0.28 mol), prepared as described in the previous examples, were suspended into 1000 ml of dichloromethane and 117,2 g of triethylamine (1.16 mol) were added. The reaction mixture was cooled to 0°C-5°C and 121.8 g of trifluoroacetic anhydride (0.58 mol) were gradually added in an hour keeping the temperature at 5°C - 10°C. At the end of the addition, the reaction mixture was kept in such condition for 5 hours. The reaction mixture was diluted with 300 ml of water, the layers were separated and the organic layer was washed with 1 x 100 ml of a saturated solution of sodium bicarbonate and with 3 x 150 ml of water. The combined organic layers were concentrated up to a residue under vacuum distillation to afford 85 g of N-formyl-N-(3-trifluoroacetyl-1-adamantyl)acetyl-pyrrolidine-2-nitrile.
1 H-NMR (CDCl₃, 300 MHz): δ 8.54 (s, 1 H), 4.70 (m, 1 H), 4.07 (m, 2H), 3.60 (m, 2H), 2.19 (s, 2H), 2.13 (s, 2H), 1.88 (m, 2H), 1.80 (s, 6H), 1.55 (s, 4H), 1.41 (s, 2H).
ESI/MS (MH⁺): 429

### Example 6

In a reaction flask under nitrogen flow, 20.4 g of N,N-carbonyldiimidazole (0.126 mol) and 175 ml of tetrahydrofurane were added at 25°C, then 26.8 g of N-formyl-N-(3-hydroxy-1-adamantyl)aminoacetic acid (0.106 mol) prepared as described in example 1 or 2, were gradually added and the suspension was kept at these conditions till complete solubilization. Then 12,9 g of L-prolinamide (0.113 mol) were added and the solution was heated at 40 °C. After the completion of reaction, a solution of 50.6 ml of ethylnicotinate (0.370 mol) in 150 ml of tetrahydrofurane were added at 25°C to the resultant suspension; then 51.5 ml of trifluoroacetic anhydride were added at 0°C and, after the addition, the reaction mixture was kept at 25°C for 1 hour. The solvent was removed by vacuum distillation till obtaining an oily residue to which 320 ml of toluene and 130 ml of water were added. The residue was the washed with a solution of 13 ml of hydrochloric acid 37 % in 130 ml of water, 3 x 130 ml of water and 130 ml of brine. The organic layer was filtered on Celite ® pad and the solvent was removed under vacuum to afford 40 g of N-formyl-N-(3-trifluoroacetyl-1-adamantyl)acetyl-pyrrolidine-2-nitrile as an oily residue.
1 H-NMR (CDCl₃, 300 MHz): δ 8.54 (s, 1 H), 4.70 (m, 1 H), 4.07 (m, 2H), 3.60 (m, 2H), 2.19 (s, 2H), 2.13 (s, 2H), 1.88 (m, 2H), 1.80 (s, 6H), 1.55 (s, 4H), 1.41 (s, 2H).
ESI/MS (MH⁺): 429

### Example 7

In a reaction flask 100 ml of water, 39.4 g of potassium carbonate and 10 ml of methanol were added to N-formyl-N-(3-trifluoroacetyl-1-adamantyl)acetyl-pyrrolidine-2-nitrile, prepared as described in examples 5 or 6.

The resultant reaction mixture was heated to 40°C for 4 hours. At the end of the hydrolysis reaction, the mixture was distilled under vacuum to afford 76 g of N-formyl-N-(3-hydroxy-1-adamantyl)acetyl-pyrrolidine-2-nitrile as an oily residue.
¹H-NMR (CDCl₃, 300 MHz): δ 8.25 (s, 1 H), 4.24 (t, 1 H), 4.09 (s, 2H), 3.53 (t, 2H), 2.19 (s, 2H), 2.13 (s, 2H), 1.88 (m, 2H), 1.80 (s, 6H), 1.55 (s, 4H), 1.41 (s, 2H)
¹³C-NMR (CDCl₃, 300 MHz): δ 169.5 (CH), 119.3 (C), 71.6 (C), 62.4 (CH), 61.5 (C), 49.4 (CH₂), 48.9 (CH₂), 44.4 (CH₂), 44.2 (CH₂), 41.2 (CH₂), 35.6 (CH₂), 32.1 (CH), 31.4 (CH₂), 26.3 (CH₂)
ESI/MS (MH⁺): 332

### Example 8

In a reaction flask under nitrogen flow, 100 g of N-formyl-N-(3-hydroxy-1-adamantyl)acetyl-pyrrolidine-2-amide (0.28 mol), prepared as described in the previous examples, are suspended into 1000 ml of dichloromethane and 117.2 g of triethylamine (1.16 mol) were added. The reaction mixture was cooled to 0°C - 5°C and 121.8 g of trifluoroacetic anhydride (0.58 mol) were gradually added during an hour keeping the temperature at 5°C - 10°C. At the end of the addition the reaction mixture was kept in such conditions for 5 hours. The reaction mixture was diluted with 300 ml of water, the layers were separated and the organic layer was washed with 3 x 150 ml of water. Into the organic phase 100 ml of water, 39.4 ml of potassium carbonate and 10 ml of methanol were added; the resultant mixture was heated at 40°C for 4 hours. At the end of the hydrolysis reaction the mixture was distilled under vacuum to afford 76 g of N-formyl-N-(3-hydroxy-1-adamantyl)acetyl-pyrrolidine-2-nitrile as an oily residue.
¹H-NMR (CDCl₃, 300 MHz): δ 8.25 (s, 1 H), 4.24 (t, 1 H), 4.09 (s, 2H), 3.53 (t, 2H), 2.19 (s, 2H), 2.13 (s, 2H), 1.88 (m, 2H), 1.80 (s, 6H), 1.55 (s, 4H), 1.41 (s, 2H)
¹³C-NMR (CDCl₃, 300 MHz): δ 169.5 (CH), 119.3 (C), 71.6 (C), 62.4 (CH), 61.5 (C), 49.4 (CH₂), 48.9 (CH₂), 44.4 (CH₂), 44.2 (CH₂), 41.2 (CH₂), 35.6 (CH₂), 32.1 (CH), 31.4 (CH₂), 26.3 (CH₂)
ESI/MS (MH⁺): 332

### Example 9

In a reaction flask equipped with stirrer, thermometer and condenser, 100 g of N-formyl-N-(3-hydroxy-1-adamantyl)acetyl-pyrrolidine-2-nitrile (0.30 mol), prepared as described in the previous examples, were added with 750 ml of water and 36.6 g of hydrochloric acid 37 % (0.37 mol); the reaction mixture was heated to 70°C for 7 hours. The mixture was then cooled at room temperature and extracted with 1 x 100 ml of methyl-tert-butyl ether. The aqueous solution was neutralized with soda 50 %, then water was distilled under vacuum obtaining a solid to which 1000 ml of dichloromethane were added and the resultant mixture was stirred for 30 minutes. The sodium chloride resulted from the neutralization reaction was removed by filtration and, after a vacuum distillation step, a solid residue was obtained and crystallized from methylethylketone, dried in vacuum at 40°C to afford 72 g of vildagliptin.

### Example 10

In a reaction flask equipped with stirrer, thermometer and condenser, 100 g of N-formyl-N-(3-trifluoroacetyl-1-adamantyl)acetyl-pyrrolidine-2-nitrile (0.234 mol), 300 ml of water and 34.6 g of hydrochloric acid 37 % (0.35 mol) were added and the mixture was heated at 80°C for 3 hours. After the reaction completion the mixture was cooled to room temperature and soda 50 % was added up to pH 9, keeping the mixture at 25 °C. To the solution, 91 g of sodium chloride and 1000 ml of dichloromethane were added and the mixture was stirred for 30 min. The organic layer was distilled under vacuum obtaining an oily residue which was crystallized from methylethylketone, dried in vacuum at 40 °C to afford vildagliptin.

## Claims

1. A process for the synthesis of vildagliptin comprising the following steps:
a)the reaction of 1-amino-3-adamantanol (III) with glyoxylic acid to give the intermediate of formula 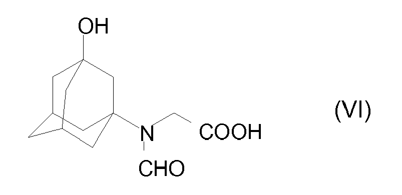
b)the reaction of intermediate (VI) with L-prolinamide to give the intermediate of formula 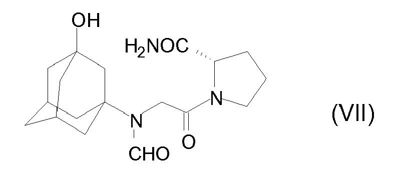
c)the dehydration of intermediate (VII) to give the corresponding nitrile derivative of formula 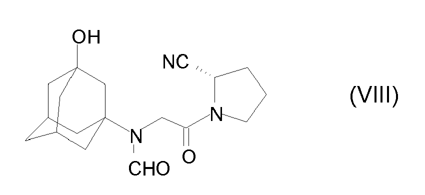
d)the deformylation of intermediate (VIII) to give vildagliptin (I).

2. A process according to claim 1 wherein step a) is carried out in a suitable solvent at a temperature from 0°C to 100°C.

3. A process according to claim 1 wherein step b) is carried out in the presence of a condensing agent and of an aprotic solvent.

4. A process according to claim 1 wherein step c) is carried out by using a suitable dehydrating agent, such as an anhydride, in the presence of a base in an inert organic solvent.

5. A process according to claim 4 wherein trifluoroacetic anhydride in the presence of triethylamine in dichloromethane is used.

6. A process according to claim 1 wherein step d) is carried out by treatment at warm with an acid followed by the optional treatment with a base.

7. A compound of formula

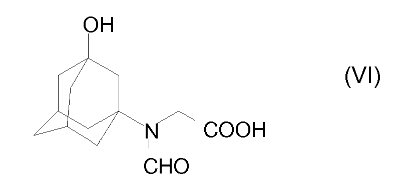

8. A compound of formula

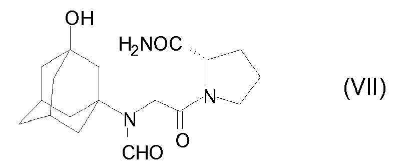

9. A compound of formula

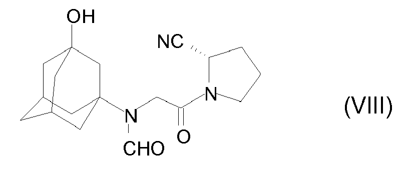

10. A compound of formula

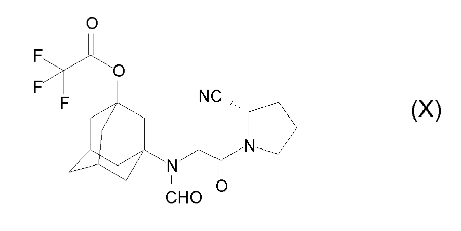

## Patentansprüche

1. Verfahren für die Synthese von Vildagliptin, umfassend die folgenden Schritte:
a) die Reaktion von 1-Amino-3-adamantanol (III) mit Glyoxylsäure, um das Zwischenprodukt der Formel 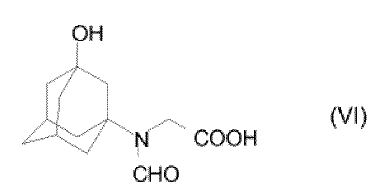 zu ergeben,
b) die Reaktion des Zwischenprodukts (VI) mit L-Prolinamid, um das Zwischenprodukt der Formel 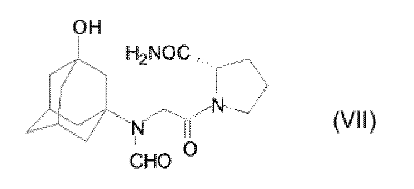 zu ergeben,
c) die Dehydratisierung des Zwischenprodukts (VII), um das entsprechende Nitrilderivat der Formel 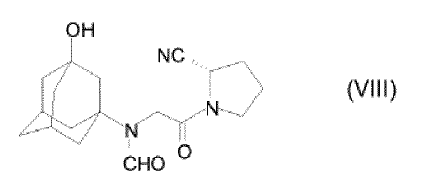 zu ergeben,
d) die Deformylierung des Zwischenprodukts (VIII), um Vildagliptin (I) zu ergeben.

2. Verfahren nach Anspruch 1, wobei Schritt a) in einem geeigneten Lösungsmittel bei einer Temperatur von 0 °C bis 100 °C durchgeführt wird.

3. Verfahren nach Anspruch 1, wobei Schritt b) in Gegenwart eines Kondensiermittels und eines aprotischen Lösungsmittels durchgeführt wird.

4. Verfahren nach Anspruch 1, wobei Schritt c) durch Verwenden eines geeigneten Dehydratisierungsmittels, wie beispielsweise eines Anhydrids, in Gegenwart einer Base in einem inerten organischen Lösungsmittel durchgeführt wird.

5. Verfahren nach Anspruch 4, wobei Trifluoressigsäureanhydrid in Gegenwart von Triethyethylamin in Dichlormethan verwendet wird.

6. Verfahren nach Anspruch 1, wobei Schritt d) durch Behandlung bei Wärme mit einer Säure, gefolgt von der wahlweisen Behandlung mit einer Base durchgeführt wird.

7. Verbindung der Formel

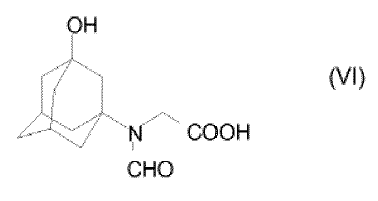

8. Verbindung der Formel

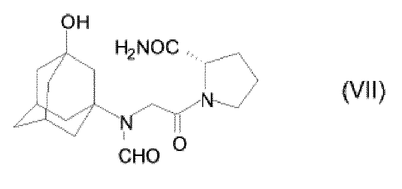

9. Verbindung der Formel

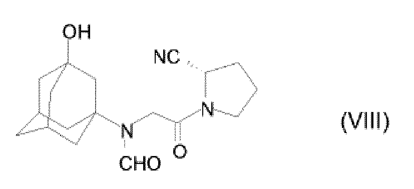

10. Verbindung der Formel

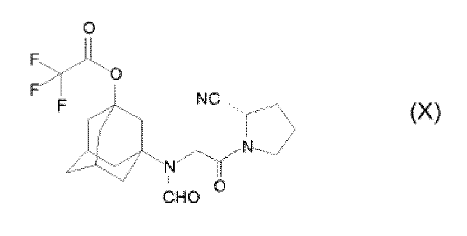

## Revendications

1. Procédé de synthèse de vildagliptine comprenant les étapes consistant à :
a) faire réagir du 1-amino-3-adamantanol (III) avec de l'acide glyoxylique pour donner le composé intermédiaire de formule : 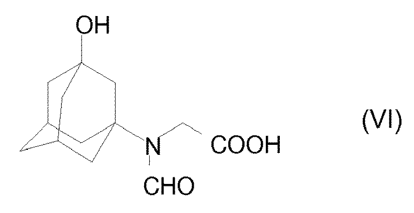
b) faire réagir le composé intermédiaire (VI) avec du L-prolinamide pour donner le composé intermédiaire de formule : 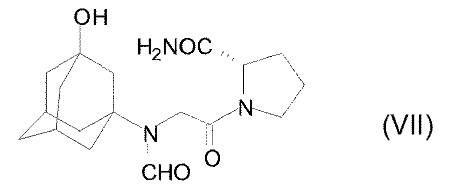
c) déshydrater le composé intermédiaire (VII) pour donner le dérivé de nitrile correspondant de formule : 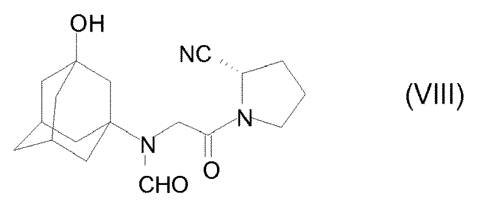
d) déformyler le composé intermédiaire (VIII) pour donner la vildagliptine (I).

2. Procédé selon la revendication 1, dans lequel l'étape a) est effectuée dans un solvant approprié à une température de 0 °C à 100 °C.

3. Procédé selon la revendication 1, dans lequel l'étape b) est effectuée en présence d'un agent de condensation et d'un solvant aprotique.

4. Procédé selon la revendication 1, dans lequel l'étape c) est effectuée en utilisant un agent déshydratant approprié, tel qu'un anhydride, en présence d'une base dans un solvant organique inerte.

5. Procédé selon la revendication 4, dans lequel on utilise de l'anhydride trifluoracétique en présence de triéthylamine dans du dichlorométhane.

6. Procédé selon la revendication 1, dans lequel l'étape d) est effectuée par traitement à chaud avec un acide suivi d'un traitement facultatif avec une base.

7. Composé de formule :

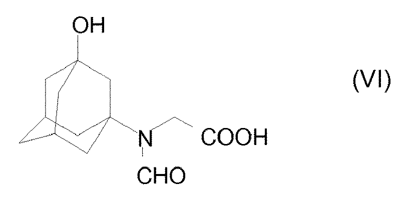

8. Composé de formule :

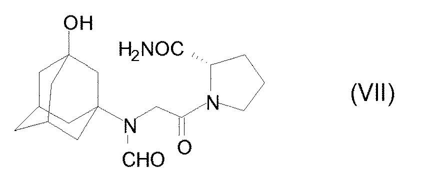

9. Composé de formule :

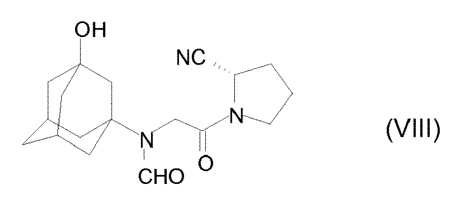

10. Composé de formule :
